# EUROPEAN PATENT APPLICATION

(11) **EP 1 892 235 A1**
(43) Date of publication of application: **27.02.2008**
(21) Application number: 06119471.8
(22) Date of filing: 24.08.2006
(51) Int. Cl.: C07C 213/00, C07C 217/08

(54) **Method for preparing arylpoly(oxyalkyl) amines**

(71) Applicant: Dishman Pharmaceuticals & Chemicals Ltd., Navrangpura, 380 009 Ahmedabad (IN)
(72) Inventor: Rajnikant Vyas, Janmejay, London, W1H 5LP (GB); Shah, Jayshree H., IN-380005, Ahmedabad (IN); Natavarlal Dave, Rakesh, IN-382 346, Ahmedabad (IN)
(74) Representative: van Westenbrugge, Andries

(57) **Abstract**

The invention pertains to a process for preparing an arylpoly(oxyalkyl)compound, wherein the process involves reacting a phenol with a halohydrin, and reacting the reaction product with an amino alkyl halide. The invention seeks to provide a less hazardous alternative to conventional preparation processes where arylpoly(oxyalkyl)compounds are made starting from an extremely toxic and hazardous dihalogeno-polyalkylene ether. The preferred arylpoly(oxyalkyl)compound is benzethonium chloride.

## Description

### TECHNICAL FIELD OF THE INVENTION

The invention pertains to the preparation of arylpoly(oxyalkyl) amines.

### BACKGROUND OF THE INVENTION

For long arylpoly(oxyalkyl) amines have shown to be excellent wetting, emulsifying, dispersing and cleaning agents which may be generally classed as capillary active compounds. Nowadays, arylpoly(oxyalkyl)amines in quaternized form are very popular for preservative in cosmetics, disinfectant in dairies and food industries, antiseptic, clinical reagent for determination of protein in CSF and as pharmaceutical aid. This is attributed to their bactericidal and fungicidal activity. Within this class of compounds, active pharmaceutical ingredients made by quatemization with benzyl chloride, so called benzethonium chloride, are particularly useful.

According to US 2,115,250, arylpoly(oxyalkyl) amines are conventionally prepared by condensing equimolecular quantities of a phenol and a polyalkylene ether, having a halogen substituent on each of its terminal carbon atoms, in the presence of an alkaline condensing agent, reacting the product thus obtained with ammonia, a primary or secondary amine and treating the resulting amine hydrohalide with an alkali or alkaline earth hydroxide. It is a complex multistep reaction sequence involving undesirable stoichiometric precipitation of sodium chloride or similar salts.

US 6,794,543 seeks to provide a novel process for preparing arylpoly(oxalkyl)-benzyldimethylammonium derivatives by reaction of arylpoly(oxyalkyl) compounds with benzyldimethylamine or substituted benzyldimethylamines, thereby overcoming the aforementioned drawbacks. However, like US 2,115,250 it still involves a first step of providing arylpoly(oxyalkyl)compounds starting from an extremely toxic and hazardous dihalogeno-polyalkylene ether. This is also the case for benzethonium chloride, where β,β'-dichlorodiethyl is used. It so happens that especially benzethonium chloride has achieved medical and pharmaceutical significance over others from this class of compounds, see for instance US 2,115,250.

Hence, there is a need in the art for an alternative preparation route for arylpoly(oxyalkyl)compounds which involves less hazardous and non-toxic reactants.

### DESCRIPTION OF THE INVENTION

It is an object of the invention to provide an alternative preparation routes for arylpoly(oxyalkyl)compounds, in particular benzethonium precursors, which avoids the use of toxic dihalogeno-polyalkylene ethers like bischloromethyl ether which is highly toxic with TLV (threshold limit value) 0.001 ppm.

It is found that these toxic compounds may be circumvented by reacting the substituted phenol starting compound subsequently with a chlorohydrin compound and an amino alkyl halide, to obtain the arylpoly(oxyalkyl)compound of interest.

The invention thus pertains to a process for preparing an arylpoly(oxyalkyl)compound of formula:

R₁-O-(CH₂)ₙ-O-(CH₂)ₙ-NR₂R₃ (I),

wherein the process involves:
a) reacting phenol R₁OH with halohydrin X₁-(CH₂)ₙ-OH; and b) reacting the reaction product with an amino alkyl halide X₂-(CH₂)ₙ-NR₂R₃,
in which
R₁ is phenyl or substituted phenyl;
n is an integer from 2 to 6, preferably n = 2;
R₂ and R₃ each independently represent hydrogen, or C₁-C₆ alkyl; and
X₁ and X₂ are each independently halogen, preferably Cl or Br.

Specifically mentioned phenolic starting compounds are phenol, cresols, thymol, carvacrol, p-tert-butyl phenol, p-sec-butyl phenol, p-tert-amyl phenol, p-sec-octyl phenol, and p-tert-octyl phenol.

The most useful arylpoly(oxyalkyl)compounds are those derived from p-alkylated phenols in which the alkyl group contains from 4 to 12 carbon atoms, since these compounds show the highest degree of activity. The alkyl group may be straight or branched chain. The most preferred reactant is p-(α,α,γ,γ-tetramethylbutyl)phenol (p-tert-octylphenol).

The halohydrin compound is preferably a chlorohydrin or bromohydrin. Preferred chlorohydrins, are 2-chloroethanol, 2-chloropropanol, 1-chloro-2-propanol, 3-chloropropanol, 4-chlorobutanol, chloro-tert-butanol. Their bromo derivatives and higher homologues may also be applied. The most preferred reactants are 2-chloroethanol and 2-bromoethanol.

The first step is preferably performed in the presence of a base. Suitable examples are sodamide, sodium methoxide, sodium hydroxide, potassium hydroxide, potassium carbonate and sodium hydride. These may be used widely in the range of 2 - 5 mol per mol phenol.

The first reaction step may advantageously involve a phase transfer catalyst (PTC). The phase transfer catalyst may be a quaternary ammonium or phosphonium salt, preferably a quaternary ammonium salt, more preferably a tetra-alkyl ammonium salt having C₂-C₈ alkyl, or benzyl-trialkylammonium salt, wherein the alkyl is C₂-C₈. The counter anion is preferably a halide or a hydrogen sulphate. Suitable examples are benzyltriethylammonium chloride, tetrabutylammonium hydrogen sulphate, tetrabutylammonium bromide. For the purpose of the invention PTC is employed in catalytic amounts, preferably in the range of 0.01 - 0.1 mol per mol of the (un)substituted phenol.

The temperature in the first step is preferably maintained at a temperature in the range of 100 - 170 °C, preferably 110 - 160 °C. Therein, the actual reaction temperature is also dependent on the specific reactants and moisture content of the mixture, which moisture determines when the reaction is complete and the salt separates. The reaction time varies between 1 - 8 hours, depending on the reactants.

The first step of the process is preferably carried out in a solvent, preferably an aprotic organic solvent, more preferably a polar solvent. Useful solvents for the first step are DMSO, DMF, sulfolane, N-methylpyrrolidone and the like, and toluene, especially DMSO and DMF.

It is preferred to treat the substituted phenol with a slight molar excess of the halohydrin compound; typically the molar ratio of substituted phenol to halohydrin is in the range of 1:1 - 1:2, especially more than 1:1.1 and less than 1:1.5.

The second step is the amination of the phenoxy alcohol. The compounds for use therein can be primary, secondary or tertiary amino halide. If a tertiary amine, it may be a X₂-(CH₂)ₙ-substituted dimethylamine, diethylamine, pyrrolidine, piperidine or morpholine, or similar structures, such as mentioned in US 2,170,111. An amine in which R₂ and R₃ are identical is preferred.

The reaction with aminoalkyl halide is preferably performed in the presence of a base. The selected base may be one or more of those listed for use in the first reaction step, and in amount of 2 - 5 mol per mol of the phenoxy alcohol obtained in the first step. Preferably caustic lye is used.

The reaction may advantageously involve a phase transfer catalyst. The phase transfer catalyst of choice and the amount thereof are similar to those reported in relation to the first step, wherein the optimum amount of PTC is in the range of 0.01 - 0.1 mol per mol phenoxy alcohol obtained in step a).

The second reaction step usually requires about 8 - 15 hours at a temperature ranging from about 80 - 150 °C, preferably 100 - 130 °C, depending on the nature and reactivity of the reactants.

The second step is also preferably carried out in a solvent. Aprotic solvents such as ketones, tertiary amides, and chloro- or methyl-substituted benzenes are preferred. Preferably, the solvent has a boiling point in the range of the preferred reaction temperature. A suitable solvent may be DMF, MIBK or toluene. Toluene is most preferred.

The product may be treated with water afterwards in order to remove polar byproducts such as salts. When water is used as the solvent in the reaction, it may be removed for the purposes of workup after the end of the reaction, for example, by azeotropic distillation, vacuum drying, spray drying or fluidized-bed drying. The crude, water-free arylpoly(oxalkyl) compound of formula (I) can be purified by high vacuum distillation.

The invention also pertains to the use of the arylpoly(oxyalkyl) compounds obtained by the above-defined process as a precursor for salts of amines or quaternary ammonium compounds, such as those disclosed in US 2,115,250. These ammonium salts are useful in many types of industry, as already addressed in the background to the invention. These are obtained by reaction of the tertiary amine of compound (I) with alkylating agents such as benzyl chloride, dimethyl sulfate, diethyl sulfate, dimethyl oxalate, methyl iodide or ethyl bromide.

Hence, in a preferred embodiment the process of the present invention further involves a step in which the arylpoly(oxyalkyl) compound is subsequently reacted with an alkylating agent, to obtain a quaternized ammonium salt.

It is particularly preferred that the arylpoly(oxyalkyl) ammonium salt is a benzethonium salt, preferably having a chloride counterion. It may be obtained from reacting (α,α,γ,γ-tetra methyl butyl) phenol with 2-chloroethanol at the conditions described above, after which the resulting amine is alkylated using benzyl chloride. Alternatively, the compound may be a methylbenzethonium chloride or arylpoly-(oxyalkyl)benzyldimethylammonium derivatives thereof, as described in US 6,794,543.

The examples which follow illustrate the invention.

### EXAMPLES

### Example 1 - benzethonium chloride precursor

### Step a)

A mixture of 206 g (1 mol) of p-(α,α,γ,γ-tetramethylbutyl)phenol (p-tert-octylphenol), more specifically para-tert-octyl phenol, was mixed with 311 g (2.25 mol) of potassium carbonate powder and 11.4 g (0.05 mol) benzyltriethylammonium chloride in N,N-dimethylformamide, which mixture was heated to 100-105 °C while vigorously stirring. Thereafter 121 g (1.5 mol) of 2-chloroethanol was added within 1-2 hours.

Then the reaction mass was heated to reflux for 3 - 5 hours while stirring continuously. The completion of the reaction was monitored by thin layer chromatography (TLC). The unreacted potassium carbonate salt was filtered off afterwards, and N,N-dimethylformamide was distilled off under reduced pressure below 100 °C. Then 500 ml purified water was charged to the residue, and the aqueous layer extracted with 2 x 300 ml toluene. The organic layer, containing the product, was washed with 2 x 500 ml saturated sodium chloride solution, while toluene was distilled off under vacuum.

The crude product 2-(4-(1,1,3,3-tetramethylbutyl)phenoxy)ethanol (p-tert-octylphenoxyethanol) was isolated as residue, a yellow to light-brown oil. Gas chromatographic (GC) purity of the product was 90 - 95 %, with a yield of 90 - 92 %. The mixture contained about 1 % unreacted para-tert-octylphenol, which did not react further with prolonged reaction time. The residual starting material did not influence the next reaction step.

### Step b)

A mixture of 250 g (1 mol) of 2-(4-(1,1,3,3-tetramethylbutyl) phenoxy)ethanol (85 - 90 % crude product of step a), 250 g (3 mol) of caustic lye and 11.4 g (0.05 mol) benzyltriethylammonium chloride in toluene was heated and water was removed using Dean and Stark water separator.

After complete removal of water, 216 g (1.5 mol) of 2-dimethylaminoethyl chloride as base (solution in toluene) was added dropwise into the reaction mass and the addition is completed within 1 - 3 hours at reflux temperature.

Finally the reaction mass was refluxed for 15 - 20 hours. The completion of the reaction was monitored by thin layer chromatography.

Then the reaction mass was cooled to room temperature and the organic layer was washed with 2 x 500 ml saturated sodium chloride solution. The toluene was distilled off under vacuum and the crude product was isolated which was a light- to dark-brown viscous oil.

The yield of the crude product was 80 - 85 % with GC purity 85 - 90%. When this crude material was distilled using high vacuum around 2 mm Hg at high temperature, it yielded material with GC purity 95 - 96 %. It was slightly yellow viscous oil, boiling at 182 - 184 °C / 2 mm Hg.

### Example 2

### Step a)

The experiment described in example 1a was repeated, wherein 108 g (1 mol) of para-cresol used instead of p-(α,α,γ,γ-tetramethylbutyl)phenol, which was mixed with 345 g (2.5 mol) of potassium carbonate powder. All other components, amounts and reaction conditions were taken the same, although 2-chlorethanol was added at a slightly faster rate, being 1 - 2 hours.

2-(p-Methyl-phenoxy)ethanol was isolated as residue which was a yellow to light brown oil. The yield of crude product was 88 - 92 %, with GC purity 90 - 95 %.

### Step b)

In step b) 152 g (1 mole) of 2-(p-methyl-phenoxy)ethanol was mixed with caustic lye and benzyltriethylammonium chloride in toluene as described in example 1b. All reaction conditions were the same.

The yield of the crude product N,N-dimethyl-2-(2-(p-methylphenoxy)ethoxy)-ethanamine was 80 - 85 %, with GC purity 85 - 90 %.

## Claims

1. A process for preparing an arylpoly(oxyalkyl)compound of formula:
R₁-O-(CH₂)ₙ-O-(CH₂)ₙ-NR₂R₃ (I),
wherein the process involves:
a) reacting phenol R¹OH with halohydrin X₁-(CH₂)ₙ-OH; and b) reacting the reaction product with an amino alkyl halide X₂-(CH₂)ₙ-NR₂R₃,
in which
R¹ is phenyl or substituted phenyl;
n is an integer from 2 to 6;
R² and R³ each independently represent hydrogen or C₁-C₆ alkyl; and
X¹ and X² are each independently halogen.

2. The process according to claim 1, wherein reaction step a) and b) are performed in the presence of a base.

3. The process according to claim 1 or 2, wherein reaction step a) and b) involve the use of a phase transfer catalyst.

4. The process according to any one of the preceding claims, wherein the molar ratio of said phenol to said halohydrin is in the range of 1:1 - 1:2.

5. The process according to any one of the preceding claims, wherein said halohydrin is 2-chloroethanol.

6. The process according to any one of the preceding claims, wherein said phenol is p-(α,α,γ,γ-tetramethylbutyl)phenol.

7. The process according to any one of the preceding claims, wherein said arylpoly(oxyalkyl)compound is subsequently reacted with an alkylating agent, to obtain a quaternized ammonium salt.

8. The process according to claim 7, wherein said alkylating agent is benzyl chloride.
